# EUROPEAN PATENT APPLICATION

(11) **EP 4 086 349 A1**
(43) Date of publication of application: **09.11.2022**
(21) Application number: 20909970.4
(22) Date of filing: 30.12.2020
(51) Int. Cl.: C12N 15/85, C12N 5/0786, A61K 35/15, A61P 37/00, G01N 33/50

(54) **COMPOSITION AND METHOD FOR INDUCING DIFFERENTIATION INTO MYELOID CELLS, AND USE THEREOF**

(30) Priority: 30.12.2019 KR 20190178516
(71) Applicant: Ulsan National Institute of Science and Technology (UNIST), Eonyang-eup Ulju-gun Ulsan 44919 (KR); Supine Therapeutics Co., Ltd., Ulsan 44919 (KR)
(72) Inventor: KIM, Jeong Beom, Uljug-un Ulsan 44919 (KR); LEE, Hyun Ah, Ulju-gun Ulsan 44919 (KR)
(74) Representative: Mewburn Ellis LLP
(86) International application number: PCT/KR2020/019444
(87) International publication number: WO 2021/137636

(57) **Abstract**

Provided are a composition and method for inducing direct conversion from a somatic cell into a myeloid cell and use thereof, in which differentiation from a somatic cell into a myeloid cell can be efficiently induced through the expression of a single direct conversion inducer without undergoing the pluripotency stage of induced pluripotent stem cells, and thus, the composition can be widely used as an effective preventive and therapeutic agent for immune diseases.

## Description

### TECHNICAL FIELD

The present disclosure relates to a composition and method for inducing direct conversion from a somatic cell into a myeloid cell, and use thereof.

### BACKGROUND ART

Existing methods for differentiation into myeloid cells using embryonic stem cells and induced pluripotent stem cells require establishing embryonic stem cells by destroying embryos, or undergoing differentiation into myeloid cells after somatic cells are reprogrammed into induced pluripotent stem cells. These conventional methods may raise ethical issues since embryonic stem cells are used. When induced pluripotent stem cells are used, a differentiation step requires time, costs, and efforts, and the artificial control of differentiation capacity is not easy, resulting in low yield, which is inefficient. In addition, it is difficult to obtain a sufficient number of cells required for drug metabolism and toxicity verification at the *in-vitro* level, and, in the application stage of cell therapy for regenerating myeloid cell functions, there is a high possibility that teratoma derived from undifferentiated cells is formed, and thus, safety problems also have arisen.

To eliminate the risk of teratoma formation as described above, research is being conducted on direct conversion of somatic cells into somatic cells of other lineages or multipotent stem cells without going through pluripotent stem cells. However, it is difficult to verify detailed mechanisms since a great number of genes are used to induce such a direction conversion process, and thus, findings have so far been insufficient. In addition, a method of producing myeloid cells by expressing only a specific gene from somatic cells has not yet been known.

Therefore, as a result of having conducted research on a method for direct conversion from a somatic cell into a myeloid cell, the inventors of the present disclosure confirmed that, when ectopic expression was induced by introducing friend leukemia (virus) integration 1 (FLI1, ETS transcription factor) as a single gene into a somatic cell, effective direct conversion of a somatic cell into a myeloid cell was possible, thus completing the present disclosure.

### DESCRIPTION OF EMBODIMENTS

### TECHNICAL PROBLEM

An objective of the present disclosure is to provide a composition for inducing direct conversion into a myeloid cell through the introduction of a specific gene into a somatic cell or the expression of a specific protein therein.

Another objective is to provide a method for direct conversion from a somatic cell into a myeloid cell *in vitro* by using the composition.

Another objective is to provide a myeloid cell induced through direct conversion by the method.

Another objective is to provide a pharmaceutical composition for the prevention or treatment of an immune disease, including the composition.

Another objective is to provide a cell therapeutic agent for the prevention or treatment of an immune disease, including the myeloid cell.

Another objective is to provide a composition for screening for a drug for the prevention or treatment of an immune disease, including the myeloid cell.

Another objective is to provide use of the composition.

Another objective is to provide a method of preventing or treating an immune disease.

### TECHNICAL SOLUTION TO PROBLEM

To achieve the above-described objectives, one aspect provides a composition for inducing direct conversion from a somatic cell into a myeloid cell, including at least one selected from the group consisting of: (1) a friend leukemia (virus) integration 1 (FLI1) protein; (2) a nucleic acid molecule encoding the protein; and (3) a vector into which the nucleic acid molecule is introduced.

The term "direct conversion" refers to a process of inducing conversion between mature (differentiated) cells of completely different cell types in higher organisms. Unlike conventional techniques that require reprogramming of somatic cells into induced pluripotent stem cells (iPSCs) and re-differentiating the iPSCs into target cells, the present disclosure differs in that direct conversion into target cells is induced without going through the stage of pluripotent stem cells such as embryonic stem cells and induced pluripotent stem cells. Currently, direct conversion is recognized for its potential applications in disease modeling, new drug development, and the like, and thus, is known to be a technique that can also be applied to gene therapy, regenerative medicine, and the like.

The composition for inducing direct conversion, according to one aspect, may include at least one selected from the group consisting of: (1) a friend leukemia virus integration 1 (FLI1) protein; (2) a nucleic acid molecule encoding the protein; and (3) a vector into which the nucleic acid molecule is introduced. In addition, the composition for inducing direct conversion, according to one aspect, may include an FLI1 protein.

In one embodiment, it can be confirmed that, by introducing, into a somatic cell, a vector including a nucleic acid molecule encoding a friend leukemia virus integration 1 (FLI1) protein, the somatic cell is directly converted into a myeloid cell.

The term "vector" is an expression vector capable of expressing a target protein in a host cell, and may refer to a gene delivery system including essential regulatory factors operably linked so that a gene insert is expressed.

The vector includes a signal sequence or a reader sequence for membrane targeting or secretion, in addition to expression regulatory factors such as a promoter, an operator, an initiation codon, a termination codon, a polyadenylation signal, and an enhancer, and may be variously constructed according to the purpose of use. The promoter of the vector may be constructive or inductive. In addition, the expression vector includes a selectable marker for selecting a host cell containing the vector, and a replicable expression vector includes an origin of replication. The vector may be self-replicating or integrated into host DNA.

In addition, the vector may be, but is not limited to, at least one vector selected from the group consisting of a plasmid vector, a cosmid vector, a viral vector, a lentiviral vector, a retrovirus vector, a human immunodeficiency virus (HIV) vector, a murineleukemia virus (MLV) vector, an avian sarcoma/leukosis (ASLV) vector, a spleen necrosis virus (SNV) vector, a rous sarcoma virus (RSV) vector, a mouse mammary tumor virus (MMTV) vector, an adenovirus vector, an adeno-associated virus vector, a Herpes simplex virus vector, and an episomal vector. In one embodiment, a lentiviral vector may be used.

The FLI1 protein may include all FLI1 proteins derived from mammals such as humans, horses, sheep, pigs, goats, camels, antelopes, and dogs. In addition, a suitable FLI1 protein may include not only a protein having the amino acid sequence of wild type thereof, but also a variant of one or more proteins selected from the group consisting of FLI1 proteins (e.g., subtypes of each protein).

The variant of the FLI1 protein refers to a protein that has a sequence different from a natural amino acid sequence of the FLI1 protein by deletion, insertion, non-conservative or conservative substitution, or a combination thereof of one or more amino acid residues in the natural amino acid sequence of the FLI1 protein, and retains the intrinsic biological functions of a wild-type protein. The variant may be a functional equivalent that exhibits the same biological activity as a wild-type protein, or a variant in which the physicochemical properties of the protein are modified as necessary, and may be a variant with enhanced structural stability in physical and chemical environments or increased physiological activity.

The FLI1 protein or a variant thereof may be isolated from nature, or recombinantly or synthetically produced (non-naturally occurring).

In addition, a nucleic acid encoding the FLI1 protein is a nucleotide sequence encoding the wild-type or variant form of the FLI1 protein as described above, and may have one or more bases mutated by substitution, deletion, insertion, or a combination thereof, and may be isolated from nature or produced using a chemical synthesis method. The nucleic acid having a nucleotide sequence encoding the FLI1 protein may be single strand or double strand, and may be DNA molecule (genome, cDNA) or RNA (mRNA) molecule.

The term "somatic cell" may refer to all cells excluding reproductive cells, and somatic cells may be derived from or isolated from, for example, mammals such as humans, horses, sheep, pigs, goats, camels, antelopes, and dogs.

The somatic cell may be, but is not limited to, at least one selected from the group consisting of a fibroblast, an epithelial cell, a muscle cell, a nerve cell, a hair cell, a hair root cell, a hair follicle cell, an oral epithelial cell, a somatic cell extracted from urine, a gastric mucosal cell, a goblet cell, a G cell, a B cell, a pericyte, an astrocyte, a blood cell, a neural stem cell, a hematopoietic stem cell, an umbilical cord blood stem cell, and a mesenchymal stem cell. In one embodiment, a fibroblast may be used.

The term "myeloid cell" refers to an intermediate cell between a promyelocyte and a metamyelocyte in the development process, as a precursor of the granulocyte lineage, and to a cell that becomes a myelocyte through a myeloblast and a promyelocyte, which then matures into a metamyelocyte, a band neutrophil, and a segmented leukocyte.

The myeloid cell may be, but is not limited to, at least one selected from the group consisting of a monocyte progenitor cell, a monocyte, a macrophage, a dendritic cell, a megakaryocyte, and a platelet. In one embodiment, the myeloid cell may be a monocyte progenitor cell.

The term "monocyte progenitor cell" refers to a type of agranular leukocyte that accounts for 4-8% of human leukocytes and is generated as a monocyte from a hematopoietic stem cell in bone marrow or spleen, and to, being a cell at a specific differentiation stage of a cell of the macrophage lineage, a cell that differentiates and matures into a monocyte through a hematopoietic stem cell, a monoblast, and a premonocyte in bone marrow, flows out into the blood, followed by migration to tissues, and differentiation into a macrophage, a histiocyte, a Langerhans cell, a dendritic cell, and the like, thereby being able to form a reticulo-endothelial system.

The term "progenitor cell" refers to a proliferative, undifferentiated parent cell that does not express a differentiation trait when a cell corresponding to a progeny is found to express a specific differentiation trait.

Also, the term "induced monocyte progenitor cell," "iMac," and "iMOP" may refer to, for example, a monocyte progenitor cell induced from a somatic cell through direct conversion according to one aspect.

Another aspect provides a method for direct conversion from a somatic cell into a myeloid cell *in vitro,* the method including bringing or inserting, into contact with or into a somatic cell, a composition including at least one selected from the group consisting of: a friend leukemia virus integration 1 (FLI1) protein; (2) a nucleic acid molecule encoding the protein; and (3) a vector into which the nucleic acid molecule is introduced.

The method for direct conversion may include: culturing a somatic cell in a medium; transforming the cultured somatic cell with a vector into which the FLI1 gene is inserted; and culturing the transformed somatic cell under culture conditions capable of inducing direct conversion.

The medium used in the culturing of the somatic cell includes all media commonly used for culturing somatic cells in the art. The medium used for culture generally contains a carbon source, a nitrogen source, and a trace element component.

In addition, the culture conditions capable of inducing direct conversion of a somatic cell into a myeloid cell may include media and/or general culture conditions commonly used to induce direct conversion of somatic cells.

Through the introduction of the composition for inducing direct conversion into a somatic cell, ectopic expression of FLI1, which is a direct conversion factor, may be induced. Ectopic expression means expression in a tissue or a cell in which a gene is not originally expressed, or expression at time different from the original expression time. According to the method of one aspect, a myeloid cell may be effectively produced from a somatic cell.

Another aspect provides a myeloid cell induced through direct conversion by bringing or inserting, into contact with or into a somatic cell, a composition including at least one selected from the group consisting of: (1) a friend leukemia virus integration 1 (FLI1) protein; (2) a nucleic acid molecule encoding the protein; and (3) a vector into which the nucleic acid molecule is introduced.

The myeloid cell induced by the composition or method according to one aspect may not express a marker specific to the derived original somatic cell and may express a myeloid cell-specific marker.

In addition, the induced myeloid cell may have a phagocytic ability and may have the ability to differentiate into an immunoreactive cell such as a macrophage.

Another aspect provides a pharmaceutical composition for the prevention or treatment of an immune disease, including the composition as an active ingredient.

The myeloid cell produced according to one aspect plays a vital role in the immune system, and thus may be applied to the prevention or treatment of diseases caused by immune diseases.

The term "prevention" refers to all actions that prevent the corresponding disease by eliminating the etiology of an immune disease or detecting early the disease.

The term "treatment" refers to all actions that alleviate or beneficially change symptoms due to an immune disease.

The immune disease that can be prevented or treated may be, for example, at least one selected from the group consisting of autoimmune disease, transplant rejection, arthritis, graft-versus-host disease, bacterial infection, sepsis, and inflammation. Specifically, the immune disease may be, but is not limited to, for example, at least one selected from the group consisting of Crohn's disease, erythema, atopy, rheumatoid arthritis, Hashimoto's thyroiditis, malignant anemia, Edison's disease, type 1 diabetes, lupus, chronic fatigue syndrome, fibromyalgia, hypothyroidism, hyperthyroidism, scleroderma, Bahcet's disease, inflammatory bowel disease, multiple sclerosis, Alzheimer's disease, Parkinson's disease, myasthenia gravis, Meniere's syndrome, Guilian-Barre syndrome, Sjogren's syndrome, vitiligo, endometriosis, psoriasis, vitiligo, systemic scleroderma, asthma, and ulcerative colitis, and may include any disease and/or pathological condition caused by decreased, lost and/or abnormal functions of the immune system.

The pharmaceutical composition may further include at least one active ingredient known to have the effect of preventing or treating an immune disease, in addition to the induced myeloid cell.

The pharmaceutical composition may further include a pharmaceutically acceptable additive. In this regard, the pharmaceutically acceptable additive may be, for example, starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, starch sodium glycolate, carnauba wax, synthetic aluminum silicate, stearic acid, magnesium stearate, aluminum stearate, calcium stearate, sucrose, or the like. The pharmaceutically acceptable additive according to the present invention may be included in an amount of 0.1 parts by weight to 90 parts by weight with respect to the pharmaceutical composition.

The pharmaceutical composition may be administered orally or parenterally in various dosage forms during actual clinical administration, and may be formulated using commonly used diluents or excipients such as fillers, extenders, binders, wetting agents, disintegrating agents, and surfactants. As suitable formulations known in the art, those disclosed in the literature (Remington's Pharmaceutical Science, the latest, Mack Publishing Company, Easton PA) may be used.

A carrier, an excipient and a diluent that may be included in the pharmaceutical composition may be lactose, dextrose, sucrose, oligosaccharides, sorbitol, mannitol, xylitol, erythritol, maltitol, starch, acacia gum, alginates, gelatin, calcium phosphate, calcium silicate, cellulose, methyl cellulose, microcrystalline cellulose, polyvinylpyrrolidone, water, methylhydroxy benzoate, propylhydroxy benzoate, talc, magnesium stearate, mineral oil, and the like.

A suitable dose of the pharmaceutical composition may vary depending on conditions and body weights of individuals, severity of disease, types of drugs, administration route, and administration time, but may be appropriately selected by those of ordinary skill in the art. To obtain desired effects, the myeloid cell induced through direct conversion according to one aspect may be administered in an amount of, for example, about 1,000 cells/time to about 10,000 cells/time, about 1,000 cells/time to about 100,000 cells/time, about 1,000 cells/time to about 1,000,000 cells/time, about 1,000 cells/time to about 10,000,000 cells/time, about 1,000 cells/time to about 100,000,000 cells/time, about 1,000 cells/time to about 1,000,000,000 cells/time, or about 1,000 cells/time to about 10,000,000,000, based on an adult patient weighing 70 kg, and may be administered once a day to several times a day at regular intervals, or may be administered several times at regular intervals.

The pharmaceutical composition may be administered to a subject via various routes. All administration methods may be predicted, and may be, for example, oral administration, rectal or intravenous administration, and intramuscular or subcutaneous injection.

The term "individual" refers to a subject with an immune disease requiring treatment and, more particularly, refers to mammals such as humans, non-human primates, mice, rats, dogs, cats, horses, and cows.

The pharmaceutical composition may be used alone for the prevention or treatment of an immune disease, or may be used in combination with surgery, radiotherapy, hormone treatment, chemotherapy, and methods using a biological response modifier.

Another aspect provides a cell therapeutic agent for the prevention or treatment of an immune disease, including the myeloid cell induced through direct conversion as an active ingredient.

The term "cell therapeutic agent" refers to a therapeutic agent using autologous, allogenic or xenogenic cells to recover the function of a tissue, and refers to a therapeutic agent used to prevent or treat an immune disease. The cell therapeutic agent including the myeloid cell obtained by induced direct conversion as an active ingredient may be used as a cell therapeutic agent for the prevention or treatment of an immune disease.

The cell therapeutic agent may further include a pharmaceutically acceptable carrier. The pharmaceutically acceptable carrier may be, for example, saline, sterile water, Ringer's solution, buffered saline, dextrose solution, maltodextrin solution, glycerol, ethanol, human serum albumin (HSA), and a mixture of two or more of these ingredients, and, if necessary, other general additives such as an antioxidant, buffer, and a bacteriostatic agent may be added.

The cell therapeutic agent may further include, for example, a diluent, a dispersing agent, a surfactant, a binder, and a lubricant, and may be formulated into an injectable formulation, for example, an aqueous solution, a suspension, or an emulsion.

Another aspect provides a composition for screening for a drug for the prevention or treatment of an immune disease, including the myeloid cell induced through direct conversion as an active ingredient.

The composition for screening for a drug for the prevention or treatment of an immune disease may be effectively used to screen for a therapeutic agent for an immune disease, as a method of identifying the reactivity of the induced myeloid cell according to one aspect in the presence or absence of a therapeutic candidate substance for an immune disease. For example, the induced myeloid cell according to one aspect may be used to evaluate toxicity or efficacy of a candidate substance as a cell important for recovery or treatment of an immune disease.

The toxicity may be evaluated according to a method of determining toxicity commonly used in the art. For example, the toxicity evaluation may be performed in the presence or absence of a therapeutic candidate substance or based on ICso of the induced myeloid cell according to one aspect. In addition, the efficacy may be evaluated in the presence or absence of a therapeutic candidate substance, according to a method, used in the art, capable of confirming that the induced myeloid cell according to one aspect has the effects of treating an immune disease, such as the regeneration of damaged immune cells, promotion of the ability to differentiate into immune cells such as a macrophage and dendritic cell, or the like.

Another aspect provides use of the composition for the preparation of a drug for the prevention or treatment of an immune disease.

Another aspect provides a method of preventing or treating an immune disease, including administering a therapeutically effective amount of the pharmaceutical composition or the cell therapeutic agent to a subject.

Redundant description will be omitted in consideration of the complexity of the present specification. Unless otherwise defined herein, terms have meanings commonly used in the technical field to which the present disclosure pertains.

### ADVANTAGEOUS EFFECTS OF DISCLOSURE

When a composition for inducing direct conversion from a somatic cell into a myeloid cell, according to an embodiment, is used, differentiation from a somatic cell into a myeloid cell can be efficiently induced without going through the pluripotency stage of induced pluripotent stem cells through the expression of a single direct conversion inducer, and thus, effective prevention and treatment of immune diseases are possible.

In addition, a myeloid cell produced by a method for inducing direction conversion from a somatic cell into a myeloid cell, according to another embodiment, has the ability to differentiate into immune cells such as macrophages and dendritic cells, and thus, immune rejection can be excluded through the production of patient-specific immune cells. Therefore, the myeloid cell may be widely used in the immune disease treatment field.

### BRIEF DESCRIPTION OF DRAWINGS

FIG. 1 illustrates a view showing a process of inducing monocyte progenitor cells, according to an embodiment (FIG. 1A) and the morphology of cells obtained in the process of inducing monocyte progenitor cells (FIG. 1B).
FIG. 2 illustrates the results of flow cytometric analysis of induced monocyte progenitor cells (iMac) according to an embodiment, mouse fibroblasts, and bone marrow-derived monocyte progenitor cells (BMDM), against CD11b and F4/80 antibodies.
FIG. 3 illustrates the results of confirming the expression of CD45, CX3CR1, CD11b, and IBA-1 after immunocytochemical staining was performed on induced monocyte progenitor cells according to an embodiment.
FIG. 4 illustrates the results of confirming the phagocytosis ability of induced monocyte progenitor cells according to an embodiment.
FIG. 5 illustrates changes in cell shape after activation of induced monocyte progenitor cells according to an embodiment (FIG. 5A) and the results of confirming changes in expression levels of immune response-related genes (FIG. 5B).
FIG. 6 illustrates changes in survival rate and body weight after transplantation of induced monocyte progenitor cells according to an embodiment or activated macrophages to an inflammatory bowel disease animal model (FIG. 6A), disease activity index (FIG. 6B), colon length (FIG. 6C), histological score (FIG. 6D), and H&E staining of intestinal tissue (FIG. 6E).

### BEST MODE

Hereinafter, the present disclosure will be described in detail with reference to experimental examples and examples to aid the understanding of the present disclosure. However, the following experimental examples and examples are illustrative purposes only and are not intended to limit the scope of the present disclosure. The examples and experimental examples of the present disclosure are provided to completely explain the present disclosure to those of ordinary skill in the art.

### Example 1. Cloning and Packaging of Lentiviral Plasmid

A transcription factor (FLI1) was amplified by a polymerase chain reaction (PCR) using cDNA of HepG2. Subsequently, the amplified FLI1 was introduced into a lentiviral vector and confirmed by sequencing. The packaging of lentivirus was performed by adding a lentiviral transfer plasmid, a packaging plasmid (psPAX2), and an envelope plasmid (VSV-G) in a ratio of 3:2:1 by using an X-tremeGENE 9 DNA transfection reagent (Roche, 06365787001). 293T cells (Thermo Fisher Scientific) at a confluency of 50% were treated with a mixture of the total volume was made to 200 µl by adding DMEM (Gibco, 10313-021), and transformed for 48 hours. Thereafter, the cell culture medium was centrifuged at 80,000 g in an ultracentrifuge for 1.5 hours, and then the lentivirus pellet obtained from the centrifuged supernatant was diluted in 1 ml of DMEM.

### Example 2. RNA Extraction and cDNA Synthesis

Total RNA of the cell lysate was extracted with RiboEX (Geneall, 301-001). RNA extraction was performed according to the manufacturer's protocol. cDNA was synthesized with M-MuLV-reverse transcriptase (NEB, M0253L) by adding 500 ng of RNA and oligo-dT primer to a total of 20 µl of the reaction mixture.

### Example 3. Immunocytochemical Staining

Cells were fixed with 4% paraformaldehyde (Tech & Innovation, BPP-9004) at room temperature for 10 minutes. The fixed cells were washed three times with Dulbecco's phosphate-buffered saline (DPBS, Corning, 21-031-CV). Subsequently, the cells were permeabilized by treatment with DBPS containing 0.1% Triton X-100 (Sigma, T9284) at room temperature for 10 minutes. The cells were washed three times with DPBS, followed by reacting with DPBS containing 4% FBS at room temperature for 1 hour, to block non-specific binding. The cells were cultured with primary antibodies for 1 hour at room temperature, and then washed three times with DPBS containing 0.05% Tween-20 (Sigma, P7949). Thereafter, the cells were treated with secondary fluorescent antibodies and incubated in the dark for 1 hour. If double staining is required, an additional blocking step was performed for 30 minutes before treatment with primary antibodies according to the above process.

### Example 4. Confirmation of Immune Response and Inflammatory Response through Quantitative Real-Time PCR

DNA-free total RNA was extracted using an RNeasy mini kit (Qiagen). A total of 500 ng of RNA per reaction was used to synthesize cDNA with SuperScript^{®} III reverse transcriptase (Invitrogen). The synthesized cDNA had a total volume of 20 µl and was used as a template by LightCycler 480 SYBR Green I Mastermix (Roche). For immune response-related genes, the experiment was repeated three times, and the genes were normalized to the housekeeping gene GAPDH. Gene expression was measured by the Ct value calculation method, and all experiments were performed according to the manufacturer's protocol.

### Example 5. Production of Inflammatory Bowel Disease (IBD) Animal Model and Cell Transplantation

A colitis animal model was produced by oral administration of dextran sulfate sodium (DSS) to 4-week-old mice. After ingestion of DSS for 5 days to induce acute colitis, induced monocyte progenitor cells or activated M1/M2 macrophages were transplanted into the colon of mice.

### Experimental Example 1. Production of Induced Monocyte Progenitor Cells by Direct Conversion Factor

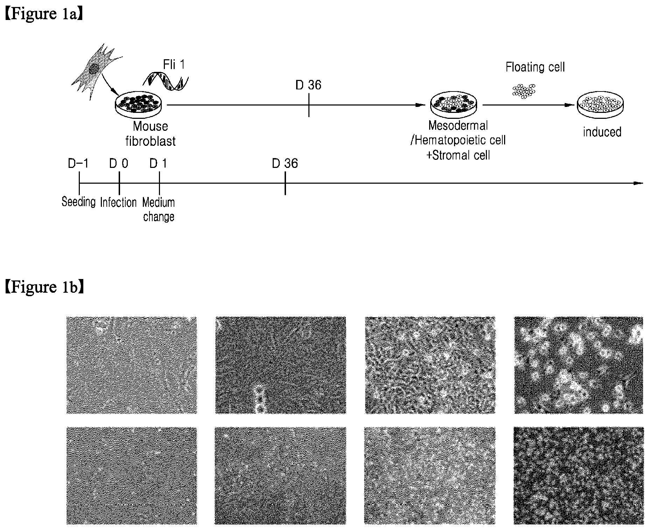

For direction conversion from somatic cells into monocyte progenitor cells, mouse fibroblasts were dispensed into a culture medium at a density of 1.5 × 10⁴ cells, and after 24 hours, the fibroblasts were transformed with the transcription factor FLI1 through the lentivirus expression system produced according to Example 1. After 24 hours, the medium composition was replaced with a monocyte progenitor cell induction medium to induce differentiation into monocyte progenitor cells. The monocyte progenitor cells induced by mechanical separation of the monocyte progenitor cell population were picked and subcultured. A schematic view showing the processes of the experiment is illustrated in FIG. 1A, and the histological morphology of cells obtained by culturing the induced monocyte progenitor cells is illustrated in FIG. 1B.

As illustrated in FIG. 1B, it was confirmed that cell aggregates were formed about 36 days after the transformation with lentivirus, and the typical morphology of monocyte progenitor cells appeared after separation and culturing. Through these results, it was confirmed that, when fibroblasts as somatic cells were treated with FLI1 to induce ectopic expression of the factors, monocyte progenitor cells could be effectively produced. In particular, it was confirmed that the monocyte progenitor cells induced by the above process showed the histological morphology shown in the actual monocyte progenitor cells.

### Experimental Example 2. Characterization of Induced Monocyte Progenitor Cells

### Experimental Example 2.1. Flow Cytometry

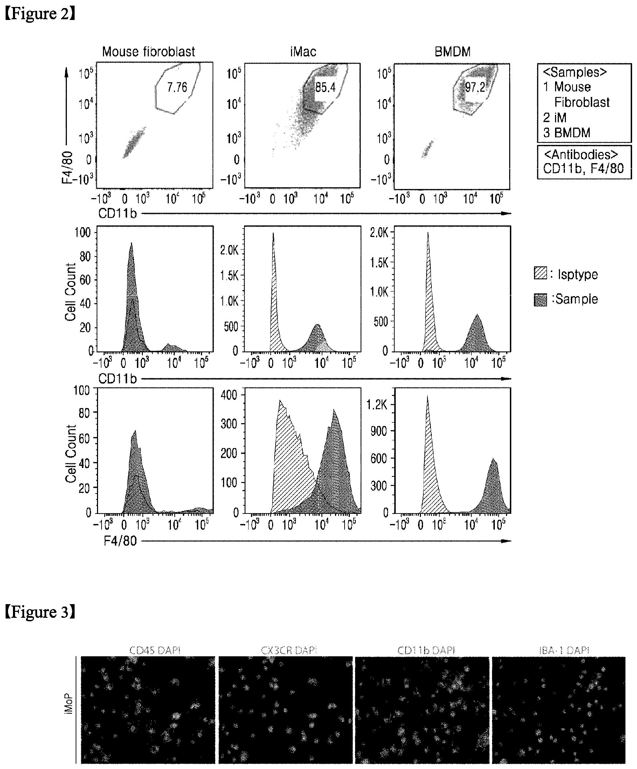

To confirm whether the monocyte progenitor cells (iMac) induced according to Experimental Example 1 show not only the histological morphology but also the characteristics of actual monocyte progenitor cells, fluorescence activated cell sorter (FACS) analysis was performed. Specifically, to confirm whether the induced monocyte progenitor cells obtained according to Experimental Example 1 express CD11b and F4/80, which are monocyte progenitor cell-specific markers, FACS analysis using CD11b and F4/80 antibodies was performed on each of fibroblasts and bone marrow-derived monocyte progenitor cells, and the results thereof are illustrated in FIG. 2.

As illustrated in FIG. 2, it was confirmed that the induced monocyte progenitor cells had a markedly different expression pattern from fibroblasts, and the monocyte progenitor cell markers CD11b and F4/80, which were not expressed in the fibroblasts, were strongly expressed in the induced monocyte progenitor cells, as in the bone marrow-derived monocyte progenitor cells. The expression levels of the CD11b and F4/80 markers in the induced monocyte progenitor cells were confirmed to be 85.4, which is similar to the expression levels thereof, i.e., 97.2, in the bone marrow-derived monocyte progenitor cells.

Through this, it was confirmed that the method described in Experimental Example 1 could efficiently induce monocyte progenitor cells.

### Experimental Example 2.2. Immunocytochemical Staining

To confirm whether the monocyte progenitor cells induced according to Experimental Example 1 express CD45, CX3CR1, CD11b, and IBA-1, which are monocyte progenitor cell-specific markers, immunocytochemical staining was performed according to the method described in Example 3 and the results thereof are illustrated in FIG. 3.

As illustrated in FIG. 3, it was confirmed that the induced monocyte progenitor cells expressed all of CD45, CX3CR1, CD11b, and IBA-1.

Through this, it was confirmed that the monocyte progenitor cells induced through direct conversion from somatic cells by using the method described in Experimental Example 1 had not only the morphology but also the actual characteristics of monocyte progenitor cells.

### Experimental Example 3. Confirmation of Phagocytosis Ability of Induced Monocyte Progenitor Cells

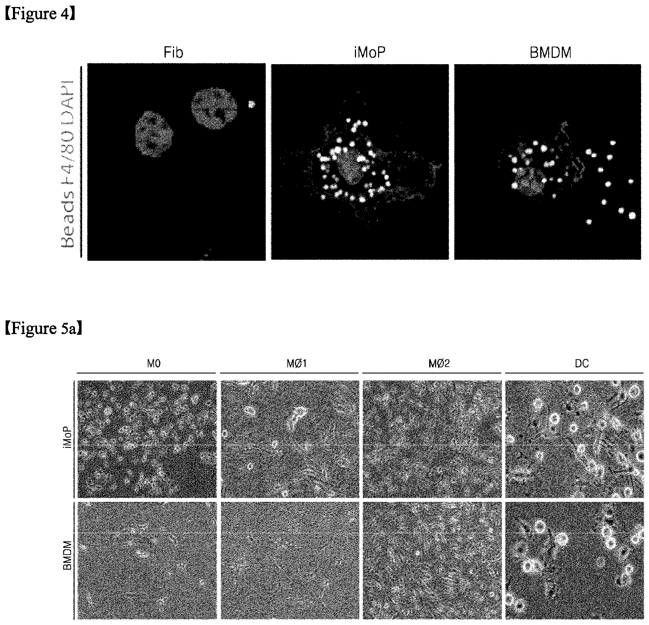

To confirm whether the monocyte progenitor cells induced according to Experimental Example 1 have the phagocytosis ability, which is a typical characteristic of immune cells, the cell culture medium was treated with green fluorescently labeled latex beads, and whether the beads are introduced into the cytoplasm through phagocytosis was confirmed with a fluorescence microscope, and the results thereof are illustrated in FIG. 4.

As illustrated in FIG. 4, green beads were introduced into the cytoplasm of the induced monocyte progenitor cells, through which it was confirmed that the induced monocyte progenitor cells had the ability to phagocytize foreign substances.

### Experimental Example 4. Confirmation of Immune Response of Induced Monocyte Progenitor Cells

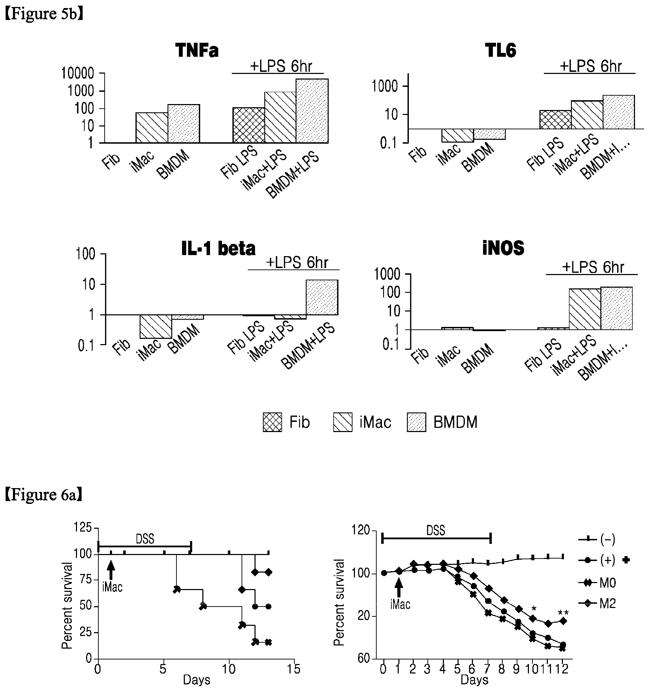

Monocyte progenitor cells are immune cells and are activated as M1 macrophages that cause an inflammatory response by foreign substances or pathogens or M2 macrophages that have anti-inflammatory action. To confirm the immune response of the monocyte progenitor cells induced according to Experimental Example 1, the induced monocyte progenitor cells were activated by treatment with each of the immune response inducers LPS, IL-4, TGFb1 and IFN-gamma or a combination thereof, and then the morphology of the cell was observed, the expression levels of immune response-related genes were confirmed using the method described in Example 4 through real-time RT-PCR analysis, and the results thereof are illustrated in FIG. 5.

As illustrated in FIG. 5, it was confirmed that the activated monocyte progenitor cells exhibited the flat amoeba-shaped or rod-shaped cell morphology characteristic of phagocytic cells, and the expression of genes involved in inflammation or anti-inflammatory action was increased.

### Experimental Example 5. Confirmation of In-vivo Immune Disease Treatment Effect of Induced Monocyte Progenitor Cells

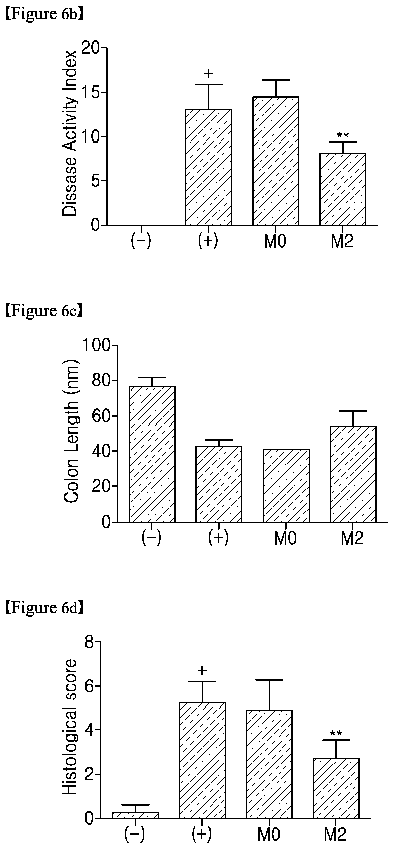

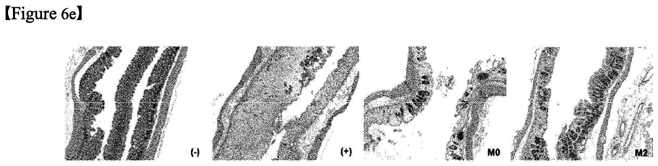

To confirm the *in-vivo* functional characteristics of the monocyte progenitor cells induced according to Experimental Example 1, the induced monocyte progenitor cells were transplanted into mice of the inflammatory bowel disease animal model produced in Example 5, changes in survival rate and body weight of the mice were analyzed, and the results thereof are illustrated in FIG.6.

As illustrated in FIG. 6, it was confirmed that the group transplanted with the induced monocyte progenitor cells exhibited a high survival rate and less weight loss. Disease activity index and maintenance of normal colon length were also improved in the group transplanted with the induced monocyte progenitor cells.

## Claims

1. A composition for inducing direct conversion from a somatic cell into a myeloid cell, the composition comprising at least one selected from the group consisting of:
(1) a friend leukemia (virus) integration 1 (FLI1, ETS transcription factor) protein;
(2) a nucleic acid molecule encoding the protein; and
(3) a vector into which the nucleic acid molecule is introduced.

2. The composition of claim 1, wherein the myeloid cell comprises at least one selected from the group consisting of a monocyte progenitor cell, a monocyte, a macrophage, a dendritic cell, a megakaryocyte, and a platelet.

3. The composition of claim 1, wherein the somatic cell comprises at least one selected from the group consisting of a fibroblast, an epithelial cell, a muscle cell, a nerve cell, a hair cell, a hair root cell, a hair follicle cell, an oral epithelial cell, a somatic cell extracted from urine, a gastric mucosal cell, a goblet cell, a G cell, a B cell, a pericyte, an astrocyte, a blood cell, a neural stem cell, an oligodendrocyte progenitor cell, a hematopoietic stem cell, an umbilical cord blood stem cell, and a mesenchymal stem cell.

4. The composition of claim 1, wherein the vector comprises at least one selected from the group consisting of a plasmid vector, a cosmid vector, a viral vector, a lentiviral vector, a retrovirus vector, a human immunodeficiency virus (HIV) vector, a murineleukemia virus (MLV) vector, an avian sarcoma/leukosis (ASLV) vector, a spleen necrosis virus (SNV) vector, a rous sarcoma virus (RSV) vector, a mouse mammary tumor virus (MMTV) vector, an adenovirus vector, an adeno-associated virus vector, a Herpes simplex virus vector, and an episomal vector.

5. A method for direct conversion of a somatic cell into a myeloid cell *in vitro,* the method comprising bringing or inserting, into contact with or into a somatic cell, a composition comprising at least one selected from the group consisting of:
(1) a friend leukemia virus integration 1 (FLI1) protein;
(2) a nucleic acid molecule encoding the protein; and
(3) a vector into which the nucleic acid molecule is introduced.

6. A myeloid cell induced through direct conversion by bringing or inserting, into contact with or into a somatic cell, a composition comprising at least one selected from the group consisting of:
(1) a friend leukemia virus integration 1 (FLI1) protein;
(2) a nucleic acid molecule encoding the protein; and
(3) a vector into which the nucleic acid molecule is introduced.

7. A pharmaceutical composition for the prevention or treatment of an immune disease, the pharmaceutical composition comprising the composition of claim 1 as an active ingredient.

8. The pharmaceutical composition of claim 7, wherein the immune disease comprises at least one selected from the group consisting of Crohn's disease, erythema, atopy, rheumatoid arthritis, Hashimoto's thyroiditis, malignant anemia, Edison's disease, type 1 diabetes, lupus, chronic fatigue syndrome, fibromyalgia, hypothyroidism, hyperthyroidism, scleroderma, Bahcet's disease, inflammatory bowel disease, multiple sclerosis, Alzheimer's disease, Parkinson's disease, myasthenia gravis, Meniere's syndrome, Guilian-Barre syndrome, Sjogren's syndrome, vitiligo, endometriosis, psoriasis, vitiligo, systemic scleroderma, asthma, and ulcerative colitis.

9. A cell therapeutic agent for the prevention or treatment of an immune disease, the cell therapeutic agent comprising the myeloid cell induced through direct conversion of claim 6 as an active ingredient.

10. A composition for screening for a drug for the prevention or treatment of an immune disease, the composition comprising the myeloid cell induced through direct conversion of claim 6 as an active ingredient.

11. Use of the composition according to any one of claims 1 to 4 for the preparation of a drug for the prevention or treatment of an immune disease.

12. A method for the prevention or treatment of an immune disease, the method comprising administering a therapeutically effective amount of the pharmaceutical composition of claim 7 to a subject.
